# EUROPÄISCHE PATENTSCHRIFT

(11) **EP 0 701 822 B1**
(45) Veröffentlichungstag und Bekanntmachung des Hinweises auf die Patenterteilung: **13.06.2001**
(21) Anmeldenummer: 95114187.8
(22) Anmeldetag: 09.09.1995
(51) Int. Cl.: A61L 15/58, A61L 15/62

(54) **Medicales Haftklebeband**
Medical pressure sensitive adhesive tape
Ruban adhésif à usage médical

(30) Priorität: 16.09.1994 DE 4433005
(43) Veröffentlichungstag der Anmeldung: 20.03.1996
(73) Patentinhaber: Lohmann GmbH & Co. KG, 56567 Neuwied (DE)
(72) Erfinder: Czech, Zbigniew, D-56075 Koblenz (DE); Sander, Dieter, D-56566 Neuwied (DE)
(74) Vertreter: Flaccus, Rolf-Dieter, Dr.

(56) Entgegenhaltungen:
- EP-A- 0 352 442
- EP-A- 0 394 956
- WO-A-92/04418
- DE-A- 4 126 230

## Beschreibung

Die Erfindung betrifft ein sterilisierbares Haftklebeband, insbesondere zur Verwendung im Operationsraum, mit einer wasserlöslichen Haftklebemasse und einem wasserdispergierbaren oder wasserlöslichen Träger.

Derartige Haftklebebänder sind prinzipiell als sogenannte redispergierbare Spleißbänder bekannt. Wasserlöslich ist eigentlich nur die Haftklebemasse, während der Träger bei mechanischer Einwirkung im Wasser redispergiert. Die Wasserlöslickeit der Haftklebemasse ist stark von den in die Polymerkette inkorporierten wasserlöslichen Monomeren und Monomeren mit hydrophilen funktionellen Gruppen abhängig. Durch eine Kombination von wasserlöslichen und hydrophilen Monomeren zeichnet sich eine aus diesen Komponenten synthetisierte Haftklebemasse durch eine vollständige Wasserlöslichkeit in alkalischem, neutralem und saurem pH-Bereich aus.
Der Stand der Technik auf diesem Gebiet wird durch folgende Patente widergespiegelt:

So zeichnen sich beispielsweise Mischungen aus carboxylgruppenhaltigen Copolymerisaten auf Isooctylacrylat/Butylacrylat-Basis und wasserlöslichen Polyoxyethylenderivaten und Phthalharztackifiern (US-A 4,413,080) durch Wasserlöslichkeit aus. Bei der Sterilisation derartiger Hatklebemassen bilden sich jedoch Abbauprodukte, die die physiologische Unbedenklichkeit gefährden können.

Wasserlösliche Haftklebemassen aus carboxylgruppenhaltigen Copolymerisaten auf Isooctylacrylat/Butylacrylat-Basis, abgemischt mit Phosporsäureestern und ethoxylierten Weichmachern (EP-B 0 141 504), besitzen Wasserlöslichkeit und lassen sich sterilisieren. Diese Haftklebemassen weisen jedoch eine unzureichende Resistenz gegenüber Hautfeuchte auf, die ihre Einsatzmöglichkeit stark einschränkt.

Wasserlösliche Haftklebemassen, aufgebaut aus Vinylphosponsäure, Acryl- oder Methacrylamid, Vinylsulfonsäure und Vinylphosponsäureestern (US-A 4,518,745) sind gegen Hautfeuchte beständig, lassen sich sterilisieren und weisen Wasserlöslichkeit auf. Aufgrund relativ hoher Konzentration an Restmonomeren ist die Hautverträglichkeit und die physiologische Unbedenklichkeit dieser Haftklebemassen eingeschränkt, so daß sie sich nicht für die Verwendung für Haftklebebänder eignen.

Der Stand der Technik gemäß DE-C 38 25 527 (wasserlösliche Haftklebemassen auf β-Acryloyloxypropionsäure-Basis) weist Schwierigkeiten bei der temporären Fixierung von textilen Materialien auf der menschlichen Haut auf, da die notwendige Haftung auf der Haut und auch auf den textilen Materialien nicht gegeben ist.

Wasserlösliche Haftklebemassen aus Acrylsäure/Acrylsäureester-Copolymeren, neutralisiert mit Alkanolaminen unter Zusatz von weichmachenden Polyoxyethylenverbindungen (US-A 3,865,770), weisen eine zu niedrige Haftung auf hydrophobierten textilen Materialien auf, was ihre praktische Anwendung bei der Herstellung der Haftklebebänder stark begrenzt.

DE-C-41 26 230 offenbart eine wasserlösliche Haftklebemasse, zusammengesetzt aus einem carboxylgruppenhaltigen Copolymerisat auf Acrylatbasis, wasserlöslichem Amin, klebrigmachendem Harz und einem carboxylgruppenhaltigen, nicht auf Acrylat basierenden Polymer, die zwar auf hydrophobierten textilen Materialien haftet, sich aber bei höheren Kleberaufträgen (>70 g/m²) nach der Dampfsterilisation braun verfärbt. Bei vielen Anwendungsgebieten setzen solche Verfärbungen die Akzeptanz stark herab.

Die Rezeptierung wasserlöslicher Haftklebemassen gemäß dem Stand der Technik berücksichtigt im übrigen auch nicht die von den Behörden für Krankenhäuser vorgeschriebenen Waschbedingungen, z.B. bei pH > 8,5 und Temperatur ca. 60 °C, denen Textilmaterialien, wie beispielsweise Abdecktücher, im hygienischen Bereich für Operationen unterworfen sind.

Der vorliegenden Erfindung liegt somit die Aufgabe zugrunde, ein Haftklebeband für den speziellen Einsatz im Hygienebreich bereitzustellen, das die oben erwähnten Mängel nicht aufweist und hautverträglich, physiologisch unbedenklich, einwandfrei sterilisierbar, hautfeuchteresistent ist und durch Dampfsterilisation nicht verfärbt wird.

Diese Aufgabe wird erfindungsgemäß gelöst durch eine Zubereitung der wasserlöslichen Haftklebemassen aus
- 30 bis 65 Gew.-% eines wasserlöslichen Polymerisats, synthetisiert aus:
   a) 60 bis 90 Gew.-% einfach ungesättigten Vinylcarbonsäuren
   b) 10 bis 40 Gew.-% Alkyl(meth)acrylaten mit 4 bis 12 C-Atomen im Alkylrest
   c) 0 bis 10 Gew.-% carboxylgruppenhaltigen (Meth)acrylamidderivaten
- 10 bis 50 Gew.-% eines wasserlöslichen Polyoxyalkylens oder eines Polyoxyalkylenderivates
- 10 bis 40 Gew.-% eines mehrfunktionellen Alkohols
- 3 bis 25 Gew.-% eines weichmachenden Harzes
- 0,1 bis 5 Gew.-% eines Vernetzungsmittels.

Einzelkomponenten der erfindungsgemäßen Haftklebemassen sind für sich alleine bekannt. Die geforderten Eigenschaften des Haftklebebandes wie Sterilisierbarkeit ohne Verfärbung, gute Haftung zu hydrophobierten textilen Operationsmaterialien und zur Haut, Hautfreuchteresistenz werden in überraschender Weise jedoch durch deren erfinderische Kombination erreicht.

Eine Ausgestaltung der Erfindung bevorzugt wasserlösliches Polymerisat auf Vinylcarbonsäure-Basis, das aus 60 bis 90 Gew.-% einfach ungesättigten Vinylcarbonsäuren, 10 bis 40 Gew.-% Alkyl(meth)acrylaten mit 4 bis 12 C-Atomen im Alkylrest und aus 0 bis 10 Gew.-% carboxylgruppenhaltigen (Meth)acrylamidderivaten synthetisiert ist.

Als einfach ungesättigte Carbonsäuren werden bevorzugt (Meth)acrylsäure, β-Acryloyloxypropionsäure, Vinylessigsäure, Fumarsäure, Crotonsäure, Aconitsäure, Dimethylacrylsäure, Trichloracrylsäure oder Itaconsäure verwendet.

Das Alkyl(meth)acrylat mit 4 bis 12 C-Atomen im Alkylrest ist vorzugsweise ein Butyl-, Pentyl-, Hexyl-, Heptyl-, Octyl-, 2-Ethylhexyl-, Isooctyl-, 2-Methylheptyl-, Nonyl-, Decyl- oder Dodecylmethacrylat.

Im Falle des carboxylgruppenhaltigen (Meth)acrylamidderivates wird beispielsweise eine (Meth)acrylamidohydroxyessigsäure, eine Bis(meth)acrylamidoessigsäure, eine 3-(Meth)acrylamido-3-methyl-butansäure oder eine 10-(Meth)acrylamidoundekansäure eingesetzt.

Die an sich schwachklebrigen, wasserlöslichen Polymere werden durch Zusatz von wasserlöslichen Weichmachern haftklebend eingestellt. Als Weichmacher können dazu verschiedene Polyoxyalkylene oder ihre Derivate verwendet werden. Die Auswahl richtet sich im wesentlichen nach der Verträglichkeit mit dem jeweiligen wasserlöslichen Polymerisat. In dieser Hinsicht haben sich wasserlösliche Polyoxyalkylene oder Polyoxyalkylenderivate einer Molmasse unter 3000 Dalton am besten bewährt. Sie werden aus der Gruppe Polyethylenglykol, Polyethylenglykolmonomethylether, Polyethylenglykolmonoethylether, Polyethlenglykoldimethylether, Polyethylenglykoldiethylether, Polypropylenglykol, Polybutylenglykol oder deren Copolymeren ausgewählt. Dabei werden zur Erzielung einer ausreichenden Haftklebrigkeit 10 bis 50 Gew.-% eines wasserlöslichen Polyoxyalkylens oder eines Polyoxyalkylenderivates benötigt.

Um beschichtete Haftklebemassen in Form von hautverträglichen, hautfeuchteresistenten und sterilisierbaren Haftklebebändern leicht und sanft von der Haut entfernen zu können, werden mehrfunktionelle Alkohole eingesetzt, wobei sich Glycerin, Ethlenglykol, Propandiol-1,2, Propandiol-1,3, Butandiol-1,2, Butandiol-1,3 oder Butandiol-1,4 in Mengen von 10 bis 40 Gew.-% als am besten geeignet erwiesen haben.

Um die Anfaßklebrigkeit (Tack) zu erhöhen, können außerdem weichmachende Harze wie beispielsweise Harze aus der Gruppe der hydrierten oder teilhydrierten Abietinsäurederivate in Anteilen von 3 bis 25 Gew.-% verwendet werden. Bei kleinen Anteilen werden sie durch die wasserlöslichen Bestandteile der Haftklebemasse fein dispers im Wasser verteilt.

Um den wasserlöslichen Haftklebemassen eine akzeptable Kohäsion zu verleihen, werden mit Carboxylgruppen des wasserlöslichen Polymerisats reagierende Vernetzungsmittel eingesetzt, wie beispielsweise Metallchelate, Metallsäureester, Epoxid-, Aziridin-, Triazin- oder Melaminformaldehydharze. Als besonders geeignet erwiesen sich Metallchelate wie Aluminium-, Hafnium-, Titan- oder Zirkoniumacetylacetonat. Von den Metallsäureestern werden Polytitansäureester bevorzugt wie z.B. Polybutyltitanat.

Als "Rückgrat"-Polymer für die Haftklebemasse wird bevorzugt ein wasserlösliches Polyacrylat verwendet, dessen Wasserlöslichkeit im alkaliscchen Bereich (pH > 8) und bei erhöhter Temperatur (oberhalb 60 °C) stark ansteigt. Eine Redispergierbarkeit des Klebebandträgers wird vorzugsweise durch Verwendung von dünnen, leicht zerfaserbaren Papieren (z.B. Teefilterpapier, Zigarettenpapier) erzielt.
Eine gute Wasserlöslichkeit stellt sich ein, wenn als Trägermaterial ein Vliesstoff verwendet wird, der wasserlösliche Fasern wie beispielsweise Calciumalginatfasern oder Polyvinylalkoholfasern enthält. Eine weitere vorteilhafte Ausführungsform der Erfindung verwendet wasserlösliche Polyvinylalkohol-, Gelatine-, Polyethylenoxid-, Stärke- oder Methylcellulose-Folie als Träger.

Das nach der Erfindung hergestellte Medical-Haftklebeband läßt sich vorteilhaft überall da einsetzen, wo neben einer vollständigen Wasserlöslichkeit hohe Hautverträglichkeit ohne Farbänderung durch Dampfsterilisation, sowie hohe spezifische Haftung auf den unterschiedlichsten textilen Flächengebilden gefordert wird, wie beispielsweise auf Baumwollmischgeweben, Baumwollgeweben, nicht imprägniert oder mit Fluorcarbonharz imprägniert, Mikrofilamentgewebe oder auf Laminaten aus Polyesterfasern/-PTFE-Membranen.
Ein bevorzugtes Anwendungsgebiet ist beispielsweise die temporäre Fixierung von Operationsabdecktüchern auf der menschlichen Haut, da die mit der Erfindung erzielten Vorteile insbesondere darin bestehen, daß neben der reinen Haftklebefunktion das Merkmal einer vollständigen Wasserlöslichkeit unter den für solche Abdecktücher typischen Waschbedingungen besteht.
Dies wird mit der Erfindung ohne Verzicht auf sonstige wichtige Eigenschaften wie Hautverträglichkeit oder Sterilisierbarkeit erreicht.
Das erfindungsgemäße Haftklebeband trägt darüber hinaus vorteilhaft zu einer Verminderung des Anteils an Einwegmaterialien im Operationsbereich bei, deren Entsorgung umweltbelastend, zeitaufwendig und teuer ist. Mit dem Haftklebeband nach der Erfindung wird somit die Ökobilanz von Operationsabdeckmaterialien entscheidend verbessert.
Ein weiteres bevorzugtes Anwendungsgebiet ist der Windelverschluß, wobei die Wiederverwertung von Baumwollwindeln durch das erfindungsgemäße Haftklebeband wesentlich gefördert wird.

Die Herstellung des wasserlöslichen Polymerisates erfolgt nach gängigen Polymersiationsverfahren, bevorzugt in Isopropanol oder in Isopropanol/Aceton-Gemisch als Lösemittel nach dem radikalischen Polymerisationsmechanismus. Als Radikalstarter werden Azoverbindungen wie 2,2'-Azobis-(2,4-dimethylpentanitril), 2,2'-Azobis-(2-methyl-propanonitril), 2,2'-Azobis-(2-methyl-butanonitril) oder 1,1'-Azobis-(cyclohexancarbonitril) eingesetzt.

Die vorliegende Erfindung wird anhand des folgenden Beispiels näher erläutert:

### Beispiel 1

200 g Copolymerisatlösung, formuliert aus 75 g Acrylsäure und 25 g Butylacrylat in 60 g Isopropylalkohol und 40 g Aceton, mit einer mittleren Molmasse von 750.000 Dalton, werden bei Raumtemperatur mit 80 g Polyethylenglykol 300, 60 g Glyzerin, 15 g Hercolyn D und 1,2 g Aluminiumacetylacetonat gemischt.

Die so erhaltene Haftklebemasse wird mittels einer Streichrakel auf eine siliconisierte 28 µm dicke Polyesterfolie aufgetragen und im Trockenkanal 10 min bei 110 °C getrocknet. Der Haftklebefilm wird beidseitig auf eine 40 µm dicke Polyvinylalkoholfolie übertragen. Die auf diesem Weg hergestellten doppelseitigen Haftklebebänder werden anschließend 25 min bei 134 °C dampfsterilisiert und nach der Dampfsterilisation einem Löslichkeitstest unterzogen.

Zur Prüfung der Wasserlöslichkeit werden 250 ml destilliertes Wasser mit Natriumhydroxid auf pH 9 eingestellt und auf 65 °C mittels eines Heizrührmotors erhitzt. Ist die Temperatur von 65 °C erreicht, werden 6,25 cm² Haftklebeband bei 400 U/min vollständig gelöst.

Ausführungsbeispiele der Erfindung werden im folgenden näher beschrieben:
Die nachfolgende Tabelle 1 enthält Polymersationszusammensetzungen, aus Tabelle 2 sind Zusammensetzungen für die Haftklebemasse des erfindungsgemäßen Haftklebebandes zusammengestellt.

Die dabei verwendeten Abkürzungen haben folgende Bedeutung:

### ABKÜRZUNGSVERZEICHNIS

- AS -: Acrylsäure
- MAS -: Methacrylsäure
- APS -: β-Acryloyloxyoropionsäure
- 2-EHA -: 2-Ethylhexylacrylat
- BA -: Butylacrylat
- NA -: Nonylacrylat
- AMHES -: Acrylamidohydroxyessigsäure
- AMUS -: 10-Acrylamidoundekansäure
- PEG 300 -: Polyethylenglykol mit einer Molmasse von 300 Dalton
- PEG 1000 -: Polyethylenglykol mit einer Molmasse von 1000 Dalton
- PEG-DME 500 -: Polyethylenglykoldimethylether mit einer Molmasse von 500 Dalton
- GC -: Glycerin
- BD-1,4 -: Butandiol-1,4
- GEDA -: Glycerinester der Dihydroabietinsäure
- C -: Weichharz Cellolyn 21
- AlACA -: Aluminiumacetylacetonat
- HfACA -: Hafniumacetylacetonat
- PBT -: Polybutyltitanat

**Tabelle 1**

| Beispiel | Vinylcarbonsäure [Gew.-%] | | | Alkyl(meth)acrylat mit bis 12 C-Atomen im Alkylrest [Gew.-%] | | | 4 Carboxylgruppenhaltiges (Meth)acrylamidderivat [Gew.-%] | |
|---|---|---|---|---|---|---|---|---|
| | AS | MAS | APS | 2-EHA | BS | NA | AMHES | AMUS |
| 2 | 80 | -- | -- | -- | 20 | -- | -- | -- |
| 3 | 60 | -- | 20 | 15 | -- | -- | 5 | -- |
| 4 | 50 | 10 | -- | -- | 20 | 15 | -- | 5 |
| 5 | -- | -- | 90 | -- | -- | 10 | -- | -- |
| 6 | -- | 75 | -- | 25 | -- | -- | -- | -- |
| 7 | 70 | -- | -- | -- | -- | 20 | 5 | 5 |
| 8 | -- | -- | 85 | 15 | -- | -- | -- | -- |
| 9 | 85 | -- | -- | -- | 10 | -- | 3 | 2 |
| 10 | -- | 60 | -- | 30 | -- | -- | 10 | -- |
| 11 | -- | -- | 80 | -- | 15 | 5 | -- | -- |
| 12 | 75 | 15 | -- | 10 | -- | -- | -- | -- |
| 13 | 65 | -- | -- | -- | 30 | -- | -- | 5 |
| 14 | 70 | -- | 10 | 20 | -- | -- | -- | -- |
| 15 | -- | 60 | 20 | -- | 20 | -- | -- | -- |

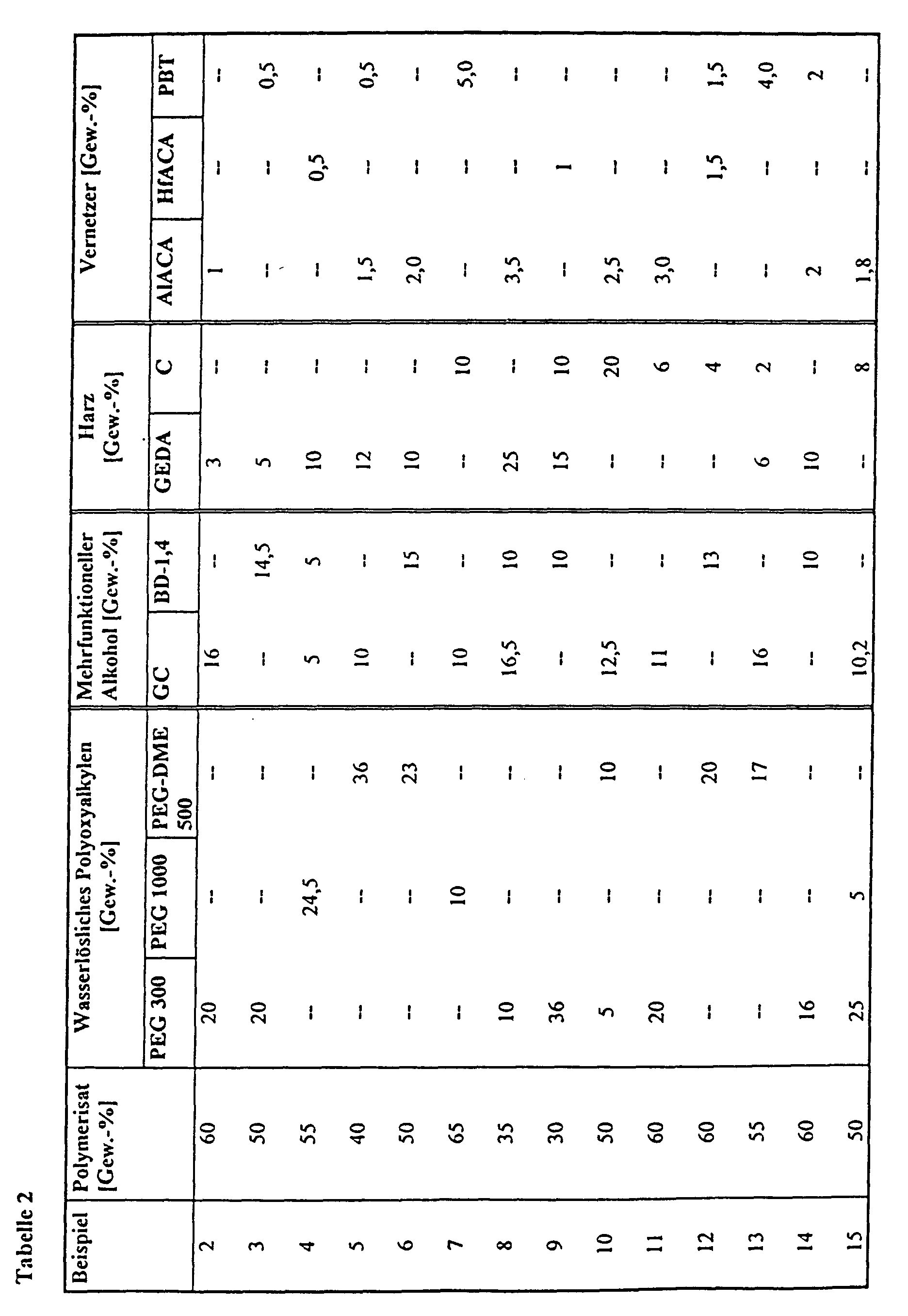

Die Haftklebemassen werden mittels einer Walzenrakel auf eine siliconisierte Polyesterfolie aufgetragen und im Trockenkanal 10 Minuten bei 100 °C getrocknet. Nach dem Trocknen werden die Haftkleberfilme beidseitig auf eine Polyvinylalkohol-Trägerfolie kaschiert. Die auf diesem Weg hergestellten doppelseitigen Medical-Klebebänder werden anschließend einem Löslichkeitstest unterzogen.
Zur Prüfung der Wasserlöslichkeit werden 250 ml destilliertes Wasser mit Natriumhydroxid auf pH 9 eingestellt und auf 65 °C mittels eines Heizrührmotors erhitzt. Ist die Temperatur erreicht, werden 6,25 cm² Klebeband bei 400 U/min gelöst. Die kompletten Klebebandsysteme lösten sich innerhalb von 10 Min. vollständig auf.
Die Ermittlung der primären Verträglichkeit der so hergestellten Klebebänder an der intakten und skarifizierten blanken Haut von Kaninchen ergab gemäß OECD-Guideline Nr. 404 die Einstufung "nicht hautreizend" (mittlerer Irritationsindex 0,0).
Auch die biologische Abbaubarkeit der wasserlöslichen Haftklebemassen ist gegeben, wie analytische Arbeiten in Anlehnung an die in den standardisierten DIN-Normen niedergelegten Verfahren aufzeigen. Danach ist die biochemische Oxidation innerhalb von 12 Tagen zu etwa 75 % abgeschlossen.

## Patentansprüche

1. Hautverträgliches, physiologisch unbedenkliches, hautfeuchteresistentes und ohne Verfärbung sterilisierbares Haftklebebeband mit einer wasserlöslichen Haftklebemasse und einem wasserlöslichen oder wasserdispergierbaren Träger, welches nach der Sterilisation auf menschlicher Haut und textilen Materialien gut haftet, dadurch gekennzeichnet, daß die wasserlösliche Haftklebemasse enthält:
- 30 bis 65 Gew.-% eines wasserlöslichen Polymerisats, synthetisiert aus:
a) 60 bis 90 Gew.-% einfach ungesättigten Vinylcarbonsäuren
b) 10 bis 40 Gew.-% Alkyl(meth)acrylaten mit 4 bis 12 C-Atomen im Alkylrest
c) 0 bis 10 Gew.-% carboxylgruppenhaltigen (Meth)acrylamidderivaten
- 10 bis 50 Gew.-% eines wasserlöslichen Polyoxyalkylens oder eines Polyoxyalkylenderivates
- 10 bis 40 Gew.-% eines mehrfunktionellen Alkohols
- 3 bis 25 Gew.-% eines weichmachenden Harzes
- 0,1 bis 5 Gew.-% eines Vernetzungsmittels.

2. Haftklebeband nach Anspruch 1, dadurch gekennzeichnet, daß das Alkyl(meth)acrylat mit 4 bis 12 C-Atomen im Alkylrest ein Butyl-, Pentyl- Hexyl-, Heptyl-, Octyl-, 2-Ethylhexyl-, Isooctyl-, 2-Methylheptyl-, Nonyl-, Decyl- oder Dodecyl(meth)acrylat ist.

3. Haftklebeband nach Anspruch 1 oder 2, dadurch gekennzeichnet, daß die einfach ungesättigte Vinylcarbonsäure aus (Meth)acrylsäure, β-Acryloyloxypropionsäure, Vinylessigsäure, Fumarsäure, Crotonsäure, Aconitsäure, Dimethylacrylsäure, Trichloracrylsäure oder Itaconsäure ausgewählt ist.

4. Haftklebeband nach einem oder mehreren der Ansprüche 1 bis 3, dadurch gekennzeichnet, daß das wasserlösliche Polyoxyalkylen oder Polyoxyalkylenderivat eine Molmasse unter 3000 Dalton besitzt und aus der Gruppe Polyethylenglykol, Polyethylenglykolmonomethylether, Polyethylenglykolmonoethylether, Polyethylenglykoldimethylether, Polyethylenglykoldiethylether, Polypropylenglykol, Polybutylenglykol oder deren Copolymeren ausgewählt ist.

5. Haftklebeband nach einem oder mehreren der Ansprüche 1 bis 4, dadurch gekennzeichnet, daß der mehrfunktionelle Alkohol aus der Gruppe Glycerin, Ethylenglykol, Propandiol-1,2, Propandiol-1,3, Butandiol-1,2, Butandiol-1,3 oder Butandiol-1,4 ausgewählt ist.

6. Haftklebeband nach einem oder mehreren der Ansprüche 1 bis 5, dadurch gekennzeichnet, daß das weichmachende Harz aus der Gruppe der hydrierten oder teilhydrierten Abietinsäurederivate ausgewählt ist.

7. Haftklebeband nach einem oder mehreren der Ansprüche 1 bis 6, dadurch gekennzeichnet, daß der Vernetzer aus der Gruppe der Metallchelate, Metallsäureeester, Epoxid-, Aziridin-, Triazin- oder Melaminformaldehydharze ausgewählt ist.

8. Haftklebeband nach einem oder mehreren der vorangehenden Ansprüche, dadurch gekennzeichnet, daß die Wasserlöslichkeit der Haftklebemasse im alkalischen Bereich (pH > 8) und bei erhöhter Temperatur (T > 65 °C) stark ansteigt.

9. Haftklebeband nach einem oder mehreren der vorangehenden Ansprüche, dadurch gekennzeichnet, daß die wasserlösliche Haftklebemasse zumindest teilweise biologisch abbaubar ist.

10. Haftklebeband nach einem oder mehreren der vorangehenden Ansprüche, dadurch gekennzeichnet, daß der Träger Papier, Vlies oder eine Folie und entweder in Wasser redispergierbar oder wasserlöslich ist.

11. Haftklebeband nach Anspruch 10, dadurch gekennzeichnet, daß der wasserlsösliche Träger ein Polyvinyl-, Polyethylenoxid-, Gelatine-, Stärke- oder eine Methylcellulose-Folie ist.

12. Verwendung des Haftklebebandes nach einem oder mehreren der vorangehenden Ansprüche für die temporäre Fixierung von textilen Materialien auf sich selbst oder auf menschlicher oder tierischer Haut.

## Claims

1. Skin-tolerable, physiologically acceptable, skin moisture-resistant pressure-sensitive adhesive tape, sterilisable without suffering a change in colour and comprising a water-soluble pressure-sensitive adhesive mass and a water-soluble or water-dispersible carrier, which adhesive tape, after sterilisation, has good adhesion to human skin and textile materials, characterised in that the water-soluble pressure-sensitive adhesive mass contains:
- 30 to 65%-wt. of a water-soluble polymer, synthesized from:
a) 60 to 90%-wt. of mono-unsaturated vinylcarboxylic acids,
b) 10 to 40%-wt. of alkyl (meth) acrylates having 4 to 12 C atoms in the alkyl residue
c) 0 to 10%-wt. of carboxyl group-containing (meth)acrylamide derivatives
- 10 to 50%-wt. of a water-soluble polyoxyalkylene or a polyoxyalkylene derivative
- 10 to 40%-wt, of a poly-functional alcohol
- 3 to 25%-wt. of a plasticizing resin
- 0.1 to 5%-wt. of a crosslinking agent.

2. Pressure-sensitive adhesive tape according to Claim 1, characterized in that the alkyl (meth) acrylate having 4 to 12 C atoms in the alkyl residue is a butyl, pentyl, hexyl, heptyl, octyl, 2-ethylhexyl, isooctyl, 2-methylheptyl, nonyl, decyl or dodecyl (meth)acrylate.

3. Pressure-sensitive adhesive tape according to Claim 1 or 2, characterized in that the mono-unsaturated vinylcarboxylic acid is (meth)acrylic acid, β-acryloyloxypropionic acid, vinylacetic acid, fumaric acid, crotonic acid, aconitic acid, dimethylacrylic acid, trichloroacrylic acid or itaconic acid.

4. Pressure-sensitive adhesive tape according to one or more of Claims 1 to 3, characterized in that the water-soluble polyoxyalkylene or polyoxyalkylene derivative has a molecular mass below 3000 Dalton and is selected from the group of polyethylene glycol, polyethyleneglycol monomethyl ether, polyethyleneglycol monoethyl ether, polyethyleneglycol dimethyl ether, polyethyleneglycol diethyl ether, polypropylene glycol, polybutylene glycol or their copolymers.

5. Pressure-sensitive adhesive tape according to one or more of claims 1 to 4, characterized in that the poly-functional alcohol is selected from the group of glycerol, ethylene glycol, propanediol-1,2, propanediol-1,3, butanediol-1,2, butanediol-1,3 or butanediol-1,4.

6. Pressure-sensitive adhesive tape according to one or more of Claims 1 to 5, characterized in that the plasticizing resin is selected from the group of the hydrogenated or partially hydrogenated abietinic acid derivatives.

7. Pressure-sensitive adhesive tape according to one or more of Claims 1 to 6, characterized in that the crosslinking agent is selected from the group of metal chelates, metal acid esters, epoxide, aziridine, triazine or melamine-formaldehyde resins.

8. Pressure-sensitive adhesive tape according to one or more of the preceding claims, characterized in that the water-solubility of the pressure-sensitive adhesive mass strongly increases in the alkaline range (pH > 8) and at increased temperature (T > 65°C).

9. Pressure-sensitive adhesive tape according to one or more of the preceding claims, characterized in that the water-soluble pressure-sensitive adhesive mass is at least partially biodegradable.

10. Pressure-sensitive adhesive tape according to one or more of the preceding claims, characterized in that the carrier is paper, nonwoven or a film or sheet, and is either redispersible in water or is water-soluble.

11. Pressure-sensitive adhesive tape according to Claim 10, characterized in that the water-soluble carrier is a film or sheet of polyvinyl, polyethylene oxide, gelatine, starch or methyl cellulose.

12. The use of the pressure-sensitive adhesive tape according to one or more of the preceding claims for temporary fixation of textile materials on themselves or on human or animal skin.

## Revendications

1. Ruban auto-adhésif compatible avec la peau, physiologiquement acceptable, résistant à l'humidité de la peau et stérilisable sans coloration, comprenant une matière auto-adhésive soluble dans l'eau et un support soluble dans l'eau ou apte à être dispersé dans l'eau, qui manifeste, après la stérilisation, une bonne adhérence à la peau humaine et à des matières textiles, caractérisé en ce que la matière auto-adhésive soluble dans l'eau contient:
- un polymère soluble dans l'eau à concurrence de 30 à 65 % en poids synthétisé à partir de:
a) des acides vinylcarboxyliques une fois insaturés à concurrence de 60 à 90 % en poids
b) des (méth)acrylates d'alkyle contenant de 4 à 12 atomes de carbone dans le radical alkyle à concurrence de 10 à 40 % en poids
c) des dérivés de (méth)acrylamide contenant des groupes carboxyle à concurrence de 0 à 10 % en poids
- un polyoxyalkylène ou un dérivé de polyoxyalkylène soluble dans l'eau à concurrence de 10 à 50 % en poids
- un alcool polyfonctionnel à concurrence de 10 à 40 % en poids
- une résine plastifiante à concurrence de 3 à 25 % en poids
- un agent de réticulation à concurrence de 0,1 à 5 % en poids.

2. Ruban auto-adhésif selon la revendication 1, caractérisé en ce que le (méth)acrylate d'alkyle contenant de 4 à 12 atomes de carbone dans le radical alkyle est un (méth)acrylate de butyle, de pentyle, d'hexyle, d'heptyle, d'octyle, de 2-éthylhexyle, d'isooctyle, de 2-méthylheptyle, de nonyle, de décyle ou de dodécyle.

3. Ruban auto-adhésif selon la revendication 1 ou 2, caractérisé en ce que l'acide vinylcarboxylique une fois insaturé est choisi parmi le groupe comprenant l'acide méthacrylique, l'acide β-acryloyloxypropionique, l'acide vinylacétique, l'acide fumarique, l'acide crotonique, l'acide aconitique, l'acide diméthylacrylique, l'acide trichloracrylique ou l'acide itaconique.

4. Ruban auto-adhésif selon une ou plusieurs des revendications 1 à 3, caractérisé en ce que le polyoxyalkylène ou le dérivé de polyoxyalkylène soluble dans l'eau possède une masse molaire inférieure à 3000 Dalton et est choisi parmi le groupe comprenant le polyéthylèneglycol, l'éther monométhylique de polyéthylèneglycol, l'éther monoéthylique de polyéthylèneglycol, l'éther diméthylique de polyéthylèneglycol, l'éther diéthylique de polyéthylèneglycol, le. polypropylèneglycol, le polybutylèneglycol ou leurs copolymères.

5. Ruban auto-adhésif selon une ou plusieurs des revendications 1 à 4, caractérisé en ce que l'alcool polyfonctionnel est choisi parmi le groupe comprenant le glycérol, l'éthylèneglycol, le propanediol-1,2, le propanediol-1,3, le butanediol-1,2, le butanediol-1,3 ou le butanediol-1,4.

6. Ruban auto-adhésif selon une ou plusieurs des revendications 1 à 5, caractérisé en ce que la résine plastifiante est choisie parmi le groupe comprenant des dérivés hydrogénés ou partiellement hydrogénés de l'acide abiétique.

7. Ruban auto-adhésif selon une ou plusieurs des revendications 1 à 6, caractérisé en ce que l'agent de réticulation est choisi parmi le groupe comprenant des chélates métalliques, des esters d'acides métalliques, des résines époxydes, d'aziridine, de triazine ou de mélamine-formaldéhyde.

8. Ruban auto-adhésif selon une ou plusieurs des revendications précédentes, caractérisé en ce que le caractère hydrosoluble de la matière auto-adhésive augmente fortement dans le domaine alcalin (pH > 8) et à température élevée (T > 65°C).

9. Ruban auto-adhésif selon une ou plusieurs des revendications précédentes, caractérisé en ce que la matière auto-adhésive soluble dans l'eau manifeste une biodégradabilité au moins partielle.

10. Ruban auto-adhésif selon une ou plusieurs des revendications précédentes, caractérisé en ce que le support est du papier; un non-tissé ou une feuille et, soit peut être remis en dispersion dans de l'eau, soit est soluble dans l'eau.

11. Ruban auto-adhésif selon la revendication 10, caractérisé en ce que le support soluble dans l'eau est une feuille de polyvinyle, une feuille d'oxyde de polyéthylène, une feuille de gélatine, une feuille d'amidon ou une feuille de méthylcellulose.

12. Utilisation du ruban auto-adhésif selon une ou plusieurs des revendications précédentes pour la fixation temporaire de matières textiles sur elles-mêmes ou sur la peau humaine ou animale.
